# EUROPEAN PATENT APPLICATION

(11) **EP 4 292 521 A1**
(43) Date of publication of application: **20.12.2023**
(21) Application number: 22853411.1
(22) Date of filing: 02.08.2022
(51) Int. Cl.: A61B 5/01, A61B 5/28, A61B 5/024, A61B 5/00, A61B 5/0205

(54) **SENSOR STRUCTURE AND ELECTRONIC DEVICE COMPRISING SENSOR STRUCTURE**

(30) Priority: 02.08.2021 KR 20210101296
(71) Applicant: Samsung Electronics Co., Ltd., Suwon-si, Gyeonggi-do 16677 (KR)
(72) Inventor: KIM, Hyuksu, Suwon-si, Gyeonggi-do 16677 (KR); LEE, June, Suwon-si, Gyeonggi-do 16677 (KR)
(74) Representative: HGF
(86) International application number: PCT/KR2022/011364
(87) International publication number: WO 2023/014034

(57) **Abstract**

An electronic device according to various embodiments disclosed herein may include a cover including a first surface and a second surface opposite to the first surface, a first electrode disposed on the first surface of the cover to be in contact with a user's body, a second electrode disposed on the second surface of the cover and electrically connected to the first electrode, a printed circuit board disposed to face the second surface of the cover, a temperature sensor electrically connected to the printed circuit board and disposed adjacent to the second electrode, and at least one processor connected to the temperature sensor, wherein the processor may be configured to generate bio-related information based on an electrical signal received through the first electrode and the second electrode, and measure the user's body temperature, which is conducted from the first electrode to the second electrode, through the temperature sensor.

## Description

### [Technical Field]

Various embodiments disclosed herein relate to a sensor structure and an electronic device including the same.

### [Background Art]

With the development of technology, wearable electronic devices having various functions have been released. A wearable electronic device may be worn on a body part (e.g., a wrist) of a user to perform various functions. Recently, wearable electronic devices including various sensors for measuring a user's physical conditions, such as a heart rate sensor or an electrocardiogram sensor, have been released.

### [Disclosure of Invention]

### [Technical Problem]

In order to measure a user's body temperature using a wearable electronic device, a heat conduction component may be disposed on a portion that comes into contact with a user's body. For example, a heat conduction component such as a metal may be disposed on a cover of the wearable electronic device that comes into contact with a user's wrist. In addition, components that measure a bioelectrical signal applied from the body may be disposed on the wearable electronic device. However, there may be an insufficient internal space to mount both a heat conduction component for body temperature measurement and a component for measuring a bioelectrical signal in a wearable electronic device.

An embodiment disclosed herein may present a structure for measuring a user's body temperature by using a component for measuring a bioelectrical signal disposed in a wearable electronic device.

### [Solution to Problem]

An electronic device according to various embodiments disclosed herein may include a cover including a first surface and a second surface opposite to the first surface, a first electrode disposed on the first surface of the cover to be in contact with a user's body, a second electrode disposed on the second surface of the cover and electrically connected to the first electrode, a printed circuit board disposed to face the second surface of the cover, a temperature sensor electrically connected to the printed circuit board and disposed adjacent to the second electrode, and at least one processor connected to the temperature sensor, wherein the processor may be configured to generate bio-related information based on an electrical signal received through the first electrode and the second electrode, and measure the user's body temperature, which is conducted from the first electrode to the second electrode, through the temperature sensor.

A sensor structure according to various embodiments disclosed herein may include a first electrode disposed on a first surface of a cover of the electronic device to be in contact with a user's body, a second electrode disposed on a second surface opposite to the first surface of the cover and electrically connected to the first electrode, a printed circuit board disposed to face the second surface of the cover, a temperature sensor electrically connected to the printed circuit board and disposed adjacent to the second electrode, and at least one processor connected to the temperature sensor, wherein the processor may be configured to generate bio-related information based on an electrical signal received through the first electrode and the second electrode, and measure the user's body temperature, which is conducted from the first electrode to the second electrode, by using the temperature sensor.

### [Advantageous Effects of Invention]

According to various embodiments disclosed herein, electrodes that come into contact with a user's body can be disposed on a wearable electronic device. A structure capable of measuring a user's bioelectrical signal and a user's body temperature using the electrodes can be presented.

### [Brief Description of Drawings]

In connection with the description of the drawings, the same or similar components may be denoted by the same or similar reference numerals.
FIG. 1 is a block diagram of an electronic device according to various embodiments in a network environment.
FIG. 2 is a front perspective view of a mobile electronic device according to various embodiments disclosed herein.
FIG. 3 is a rear perspective view of the electronic device of FIG. 2.
FIG. 4 is an exploded perspective view of the electronic device of FIG. 2.
FIG. 5A is a perspective view illustrating a state in which a sensor module according to various embodiments disclosed herein and a peripheral configuration thereof are coupled to each other.
FIG. 5B is a view illustrating a second printed circuit board illustrated in FIG. 5A and various sensors mounted on the second printed circuit board.
FIG. 6A is a cross-sectional view of a portion of the electronic device taken along line A-A of FIG. 5A.
FIG. 6B is a cross-sectional view of a portion of the electronic device taken along line B-B of FIG. 5A.
FIG. 7A is an enlarged view of an electronic component disposed in FIG. 6B.
FIG. 7B is a diagram illustrating a stacked structure of the electronic component disposed in FIG. 6B.
FIG. 8 is an exploded perspective view of an electronic device illustrating a position in which a temperature sensor module according to an embodiment is disposed on a second printed circuit board.

### [Mode for the Invention]

It should be appreciated that various embodiments of the present disclosure and the terms used therein are not intended to limit the technological features set forth herein to particular embodiments and include various changes, equivalents, or replacements for a corresponding embodiment.

With regard to the description of the drawings, similar reference numerals may be used to refer to similar or related elements. It is to be understood that a singular form of a noun corresponding to an item may include one or more of the things, unless the relevant context clearly indicates otherwise.

As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

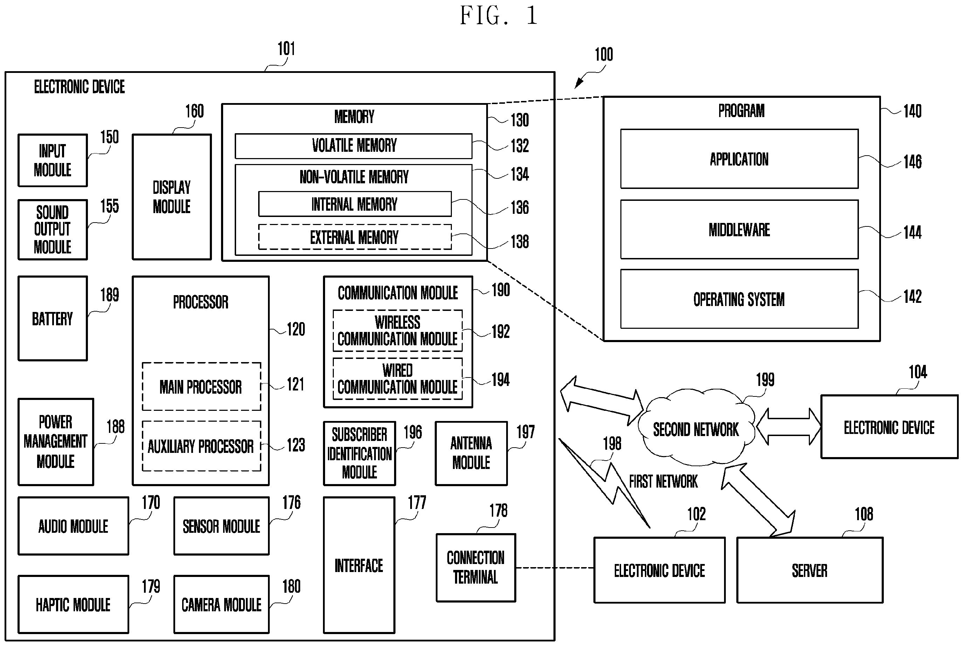

Fig. 1 is a block diagram illustrating an electronic device 101 in a network environment 100 according to various embodiments. Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adj acent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

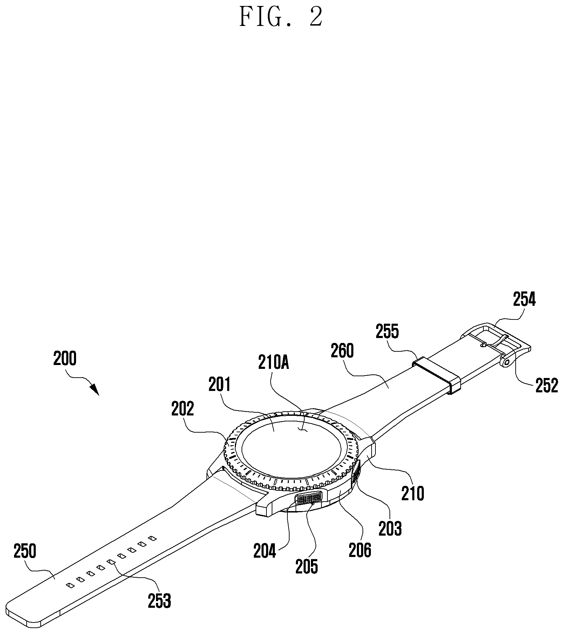

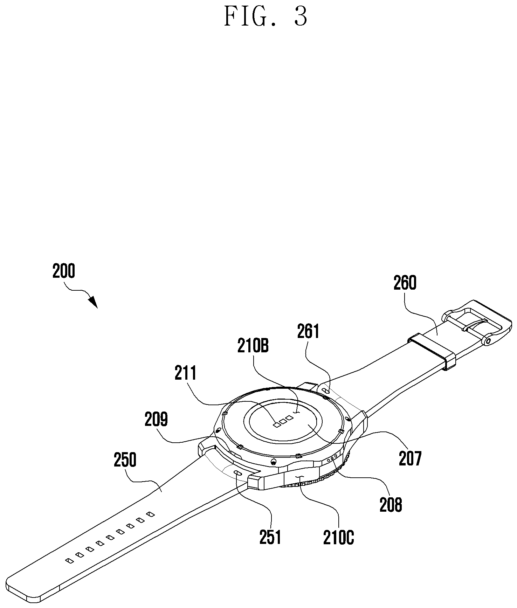

Referring to FIG. 2 and FIG. 3, the electronic device 200 according to an embodiment may include a housing 210 including a first surface (or front surface) 210a, a second surface (or rear surface) 210B, and a lateral surface 210C disposed so as to surround a space between the first surface 210a and the second surface 210B, and a coupling member 250 or 260 connected to at least a portion of the housing 210 and configured to detachably couple the electronic device 200 to a body portion (e.g.: wrist, ankle) of a user. According to another embodiment (not shown), the housing may refer to a structure for configuring a portion of the first surface 210A, the second surface 210B, and the lateral surface 210C in FIG. 2. According to an embodiment, at least a portion of the first surface 210A may be formed of substantially transparent front plate 201 (e.g.: glass plate including various coating layers or polymer plate). The second surface 210B may be formed of the substantially opaque rear plate 207. The rear plate 207 may be formed by, for example, coated or colored glass, ceramic, polymers, metals (e.g.: aluminum, stainless steel (STS), or magnesium), or a combination of at least two thereof. The lateral surface 210C may be coupled to the front plate 201 and the rear plate 207 and formed by a lateral bezel structure (or "lateral member") 206 including a metal and/or polymer. In an embodiment, the rear plate 207 and the lateral bezel structure 206 may be integrally formed and include the same material (e.g.: metal material such as aluminum). The coupling member 250 or 260 may be formed of various materials in various shapes. The coupling member may be formed to be integrally and to a plurality of unit links to be movable with each other by using a woven fabric, leather, rubber, urethane, metal, ceramic, or a combination of at least two of the materials.

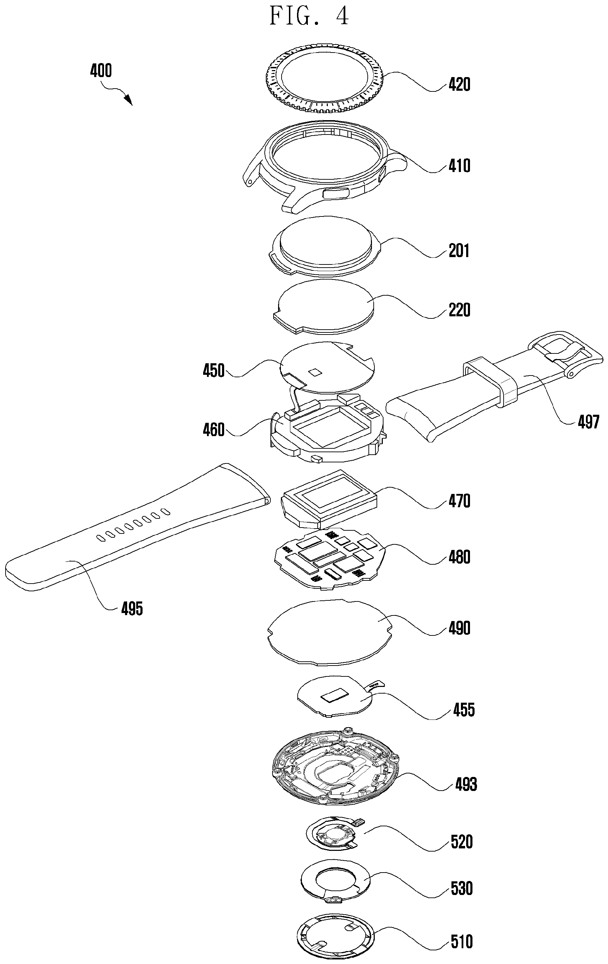

According to an embodiment, the electronic device 200 may include at least one of a display 220 (see FIG. 4), an audio module 205 or 208, a sensor module 211, a key input device 202, 203, or 204, and a connector hole 209. In an embodiment, the electronic device 200 may omit at least one of the components (e.g.: key input device 202, 203, or 204, connector hole 209, or sensor module 211) or additionally include another component.

The display 220 may be exposed to outside through, for example, a substantial portion of the front plate 201. The display 220 may have a shape corresponding to the shape of the front plate 201 in various shapes such as a circle, an oval, or a polygon. The display 220 may be combined to or disposed adjacent to a touch sensing circuit, a pressure sensor for measuring a strength (pressure) of a touch, and/or a fingerprint sensor.

The audio module 205 or 208 may include a microphone hole 205 and a speaker hole 208. A microphone for obtaining a sound from outside may be disposed in the microphone hole 205, and in an embodiment, multiple microphones may be arranged to detect a direction of a sound. The speaker hole 208 may be used as a receiver for an outer speaker and phone-calling.

The sensor module 211 may generate an electrical signal or a data value corresponding to an internal operation state or external environment state of the electronic device 200. The sensor module 211 may include, for example, a biosensor module 211 (e.g.: HRM sensor) disposed on the second surface 210B of the housing 210. The electronic device 200 may further include at least one sensor module not shown in the drawings, for example, a gesture sensor, a gyro sensor, an air pressure sensor, a magnetic sensor, an acceleration sensor, a grip sensor, a color sensor, an infrared (IR) sensor, a biometric sensor, a temperature sensor, humidity sensor, or an illuminance sensor.

The key input device 202, 203, and 204 may include a wheel key 202 disposed at the first surface 210A of the housing 210 and rotatable in at least one direction, and/or a side button key 202 or 203 disposed at the lateral surface 210C of the housing 210. The wheel key may have a shape corresponding to the front plate 202. In another embodiment, the electronic device 200 may not include a portion or entirety of the key input device 202, 203, and 204 described above, and the excluded key input device 202, 203, and 204 may be implemented as various forms such as a soft key on the display 220. The connector hole 209 may include another connector hole (not shown) capable of receiving a connector (for example, USB connector) for transmitting or receiving power and/or data to or from an external electronic device and a connector for transmitting or receiving an audio signal to or from an external electronic device. The electronic device 200 may further include, for example, a connector cover (not shown) configured to cover a portion of the connector hole 209 and block the ingress of foreign substances to the connector hole.

The coupling member 250 and 260 may be detachably coupled to at least a portion of the housing 210 by using a locking member 251 and 261. The coupling member 250 and 260 may include one or more of a fixation member 252, a fixation member fastening hole 253, a band guide member 254, and a band fixation ring 255.

The fixation member 252 may be configured to fix the coupling member 250 and 260 of the housing 210 to a body portion (e.g.: wrist and ankle) of a user. The fixation member fastening hole 253 may fix the coupling member 250 and 260 and the housing 210 to a body portion of a user by counteracting with the fixation member 252. The band guide member 254 is configured to limit the movement range of the fixation member 252 when the fixation member 252 is fastened to the fixation member fastening hole 253 so that the coupling member 250 and 260 is closely coupled to a body portion of a user. The band fixation ring 255 may limit the movement range of the coupling member 250 and 260 in a state in which the fixation member 252 is fastened to the fixation member fastening hole 253.

Referring to FIG. 4, the electronic device 400 may include a lateral bezel structure 410, a wheel key 420, a front plate 201, a display 220, a first antenna 450, a second antenna 455, a support member 460 (e.g.: bracket), a battery 470, a printed circuit board 480, a sealing member 490, a rear plate 493, and a coupling member 495 and 497. At least one of the components of the electronic device 400 may be the same as or similar to at least one of the components of the electronic device 200 in FIG. 2 or FIG. 3, and thus the overlapping description thereof will be omitted. The support member 460 may be disposed in the electronic device 400 to be connected to the lateral bezel structure 410 or integrally formed with the lateral bezel structure 410. The support member 460 may be formed of, for example, a metal material and/or a non-metal (e.g.: polymer) material. The support member 460 may have the display 220 coupled to one surface thereof and the printed circuit board 480 coupled to the other surface thereof. A processor, a memory, and/or an interface may be mounted to the printed circuit board 480. The processor may include, for example, one or more of a central processing device, an application processor, a graphic process unit (GPU), an application processor signal processing unit, or a communication processor.

The memory may include, for example, a volatile memory and a nonvolatile memory. The interface may include, for example, a high-definition multimedia interface (HDMI), a universal serial bus (USB) interface, an SD card interface, and/or an audio interface. The interface may electrically or physically connect the electronic device 400 to an external electronic device, and may include, for example, a USB connector, SD card/MMC connector, or an audio connector.

The battery 470 is a device for supplying power to at least one component of the electronic device 400, and may include, for example, a non-rechargeable primary battery, a rechargeable secondary battery, or a fuel cell. At least a part of the battery 470 may be disposed on the substantially same plane as the printed circuit board 480. The battery 470 may be integrally formed to be disposed in the electronic device 200 or may be disposed to be attachable to/detachable from the electronic device 200.

The first antenna 450 may be disposed between the display 220 and the support member 460. The first antenna 450 may include, for example, a near field communication (NFC) antenna, a wireless charging antenna, and/or a magnetic secure transmission (MST) antenna. The first antenna 450, for example, may perform a near field communication with an external electronic device, wirelessly transmit and receive power required for charging, or transmit a magnetism-based signal including a near field communication signal or payment data. In another embodiment, an antenna structure may be formed of a part or a combination of the lateral bezel structure 410 and/or the support member 460.

The second antenna 455 may be disposed between the printed circuit board 480 and the rear plate 493. The second antenna 455 may include, for example, a near field communication (NFC) antenna, a wireless charging antenna, and/or a magnetic secure transmission (MST) antenna. The second antenna 455, for example, may perform a near field communication with an external electronic device, wirelessly transmit and receive power required for charging, or transmit a magnetism-based signal including a near field communication signal or payment data. In another embodiment, an antenna structure may be formed of a part or a combination of the lateral bezel structure 410 and/or the rear plate 493.

The sealing member 490 may be disposed between the lateral bezel structure 410 and the rear plate 493. The sealing member 490 may be configured to block moisture and foreign substances from being introduced from the outside to a space surrounded by the lateral bezel structure 410 and the rear plate 493.

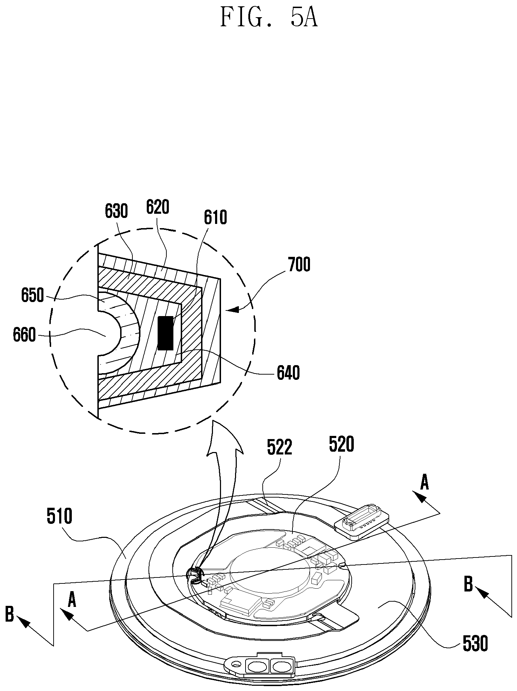

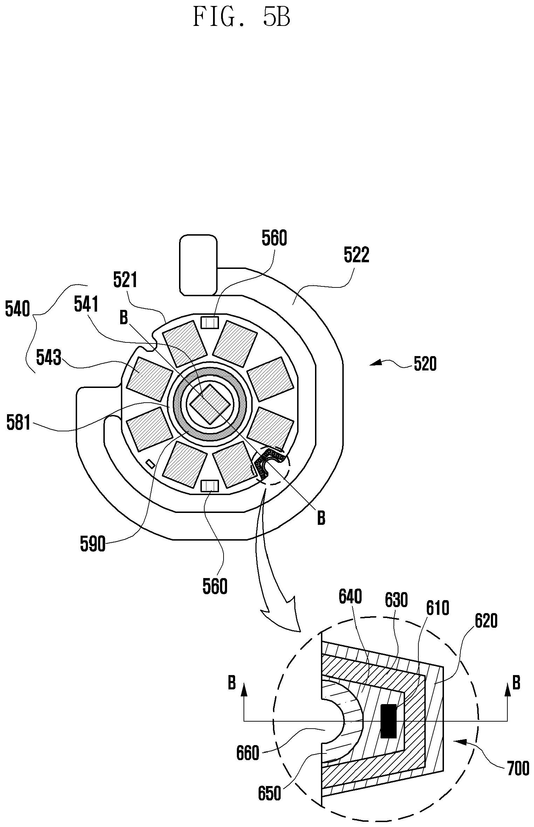

A second printed circuit board 520 (e.g., a printed circuit board (PCB), a flexible printed circuit board (FPCB), or rigid-flexible PCB (RFPCB)) and a wireless charging coil 530 may be disposed between the rear plate 493 and the rear cover 510. The second printed circuit board 520 may be electrically connected to the first printed circuit board 480 (e.g., the printed circuit board 480) via a hole provided in the rear plate 493. The wireless charging coil 530 may be disposed inside the electronic device 400. Referring to FIGS. 5A and 5B, the wireless charging coil 530 may be disposed to surround the outer periphery of the second printed circuit board 520. The wireless charging coil 530 may charge the battery 470 (e.g., the battery 189 of FIG. 1) of the electronic device 400 by receiving power from a charging device (not illustrated) outside the electronic device 400.

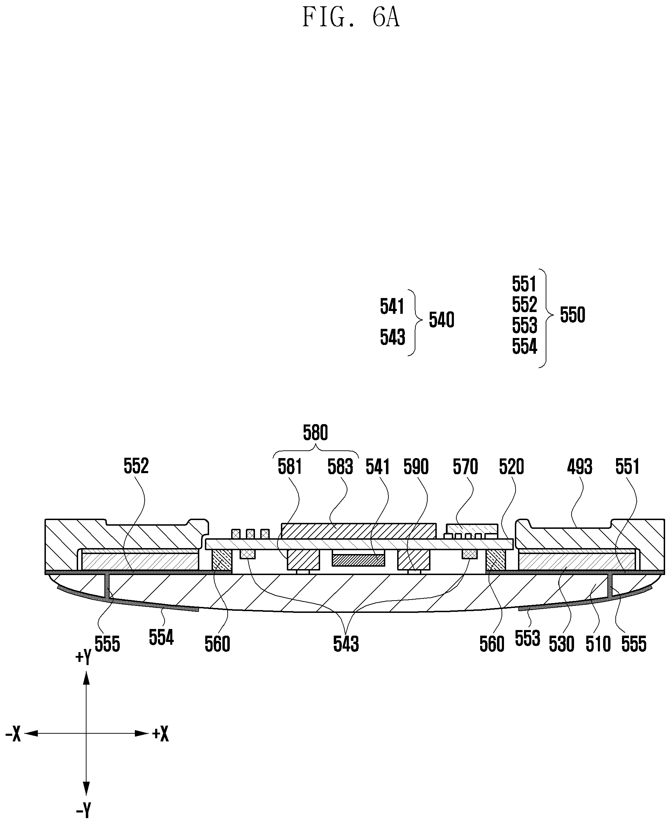

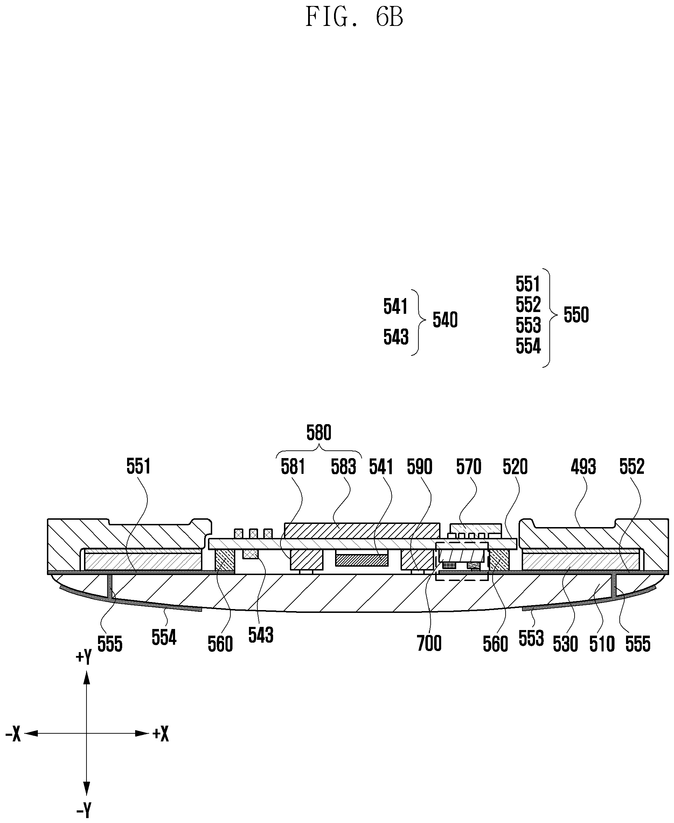

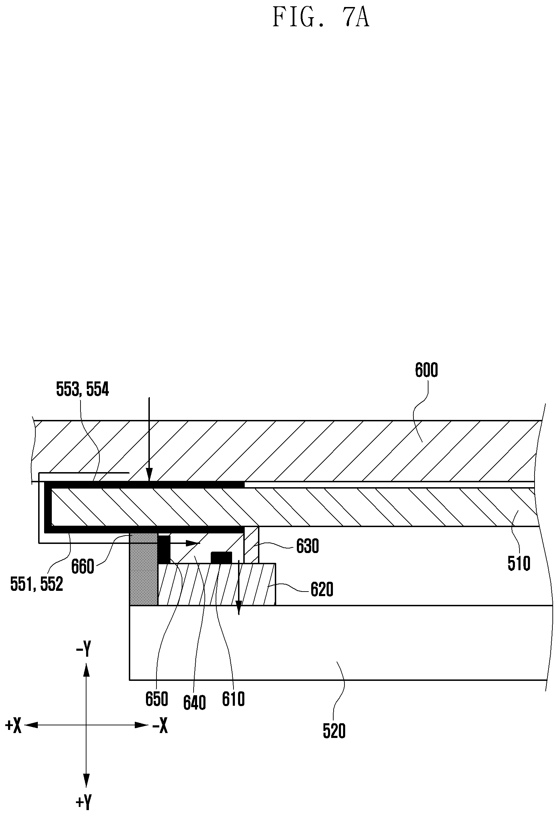

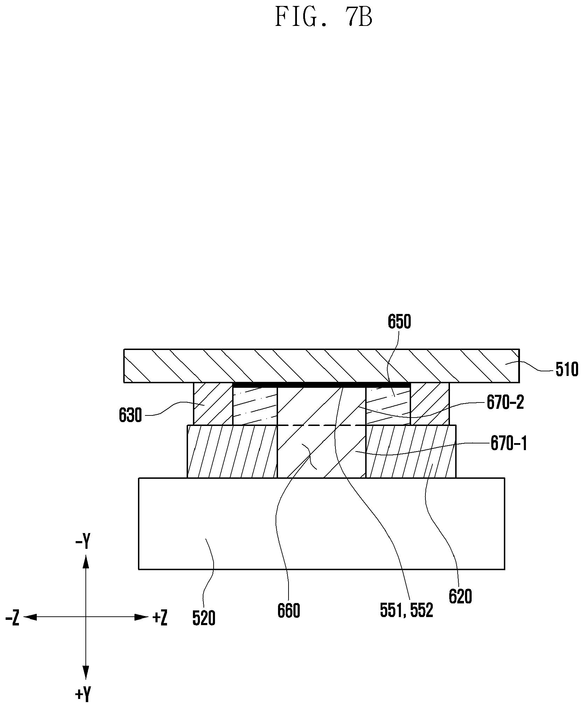

FIG. 5A is a perspective view illustrating a state in which a sensor module according to various embodiments disclosed herein and a peripheral configuration thereof are coupled to each other. FIG. 5B is a view illustrating the second printed circuit board 520 illustrated in FIG. 5A and various sensors mounted on the second printed circuit board 520. FIG. 6A is a cross-sectional view of a portion of the electronic device 400 taken along line A-A of FIG. 5A. FIG. 6B is a cross-sectional view of a portion of the electronic device 400 taken along line B-B of FIG. 5A. FIG. 7A is an enlarged view of an electronic component 700 disposed in FIG. 6B. FIG. 7B is a diagram illustrating a stacked structure of the electronic component 700 disposed in FIG. 6B.

According to various embodiments disclosed herein, the electronic device 400 (e.g., the electronic device 101 of FIG. 1, or the electronic device 200 of FIG. 2) may include a rear cover 510, a wireless charging coil 530, a second printed circuit board 520, a rear plate 493, and a first printed circuit board 480 (e.g., the printed circuit board 480 of FIG. 4). Various electronic components for detecting a user's biological signals may be disposed in the electronic device 400. For example, as will be described later, a light emitter 541 configured to detect a user's first biological signal, a light receiver 543, a contact 560, a light-tight member 590, and a temperature sensor 610 (e.g., the sensor module 176 of FIG. 1 or the sensor module 211 of FIG. 2) configured to measure the user's body temperature may be disposed on the second printed circuit board 520. External electrodes 553 and 554 and internal electrodes 551 and 552 for detecting a user's second biological signal may be disposed on the rear cover 510 disposed adjacent to the second printed circuit board 520. In addition to the above-described components, at least one electronic component may be added or deleted.

According to various embodiments, the second printed circuit board 520 may include a board body 521 on which various electronic components are mounted and a board connector 522 that electrically connects the second printed circuit board 520 to the first printed circuit board 480.

According to various embodiments, the second printed circuit board 520 may be electrically connected to the first printed circuit board 480. In an embodiment, as illustrated in FIGS. 5A and 5B, the second printed circuit board 520 may include the board connector 522. The second printed circuit board 520 may be electrically connected to the first printed circuit board 480 via the board connector 522. For example, the board connector 522 may pass through a hole provided in the center of the rear plate 493 illustrated in FIG. 4 to electrically connect the board connector 522 to the first printed circuit board 480.

According to various embodiments, a wireless charging coil 530 connected to the battery 470 (e.g., the battery 189 of FIG. 1) may be disposed on the outer periphery of the second printed circuit board 520. An induced current may be generated in the wireless charging coil 530 by a magnetic field generated by an external charging device (not illustrated), and the battery 470 may be charged by the induced current. For example, when power is supplied to the external charging device, a current flows through a coil (not illustrated) disposed inside the external charging device to generate a magnetic field. The magnetic field generated by the external charging device may generate an induced current in the wireless charging coil 530 disposed inside the electronic device 400, and the electronic device 400 may be charged by the induced current.

According to various embodiments, a magnet member 580 may be disposed on the second printed circuit board 520. The magnet member 580 may include a first magnet 581 and a second magnet 583. In an embodiment, the first magnet 581 and the second magnet 583 may have a ring shape. As illustrated in FIGS. 5B and 6A, the first magnet 581 may be disposed on a first surface (e.g., the surface oriented in the -Y direction with reference to FIG. 6A) of the second printed circuit board 520. A light emitter 541 of a first biological signal detector 540 (e.g., the sensor module 176 of FIG. 1 or the sensor module 211 of FIG. 2) may be disposed inside the first magnet 581. A light receiver 543 of the first biological signal detector 540 may be disposed on the outer periphery of the first magnet 581. The second magnet 583 may be disposed on a second surface (e.g., the surface oriented in the +Y direction with reference to FIG. 6A) opposite to the first surface of the second printed circuit board 520. The second magnet 583 may be disposed on the second surface of the second printed circuit board 520 to coincide with the central axis of the first magnet 581.

When the relative positions of the coil disposed in the external charging device and the wireless charging coil 530 are within a set range, the electronic device 400 may be wirelessly charged by the external charging device. The positions of the magnet disposed inside the external charging device and the magnet member 580 disposed inside the electronic device 400 are fixed. When the external charging device approaches the electronic device 400, an attractive force may be generated between the magnet of the external charging device and the magnet member 580 disposed in the electronic device 400. The magnet, of which the position is fixed in the external charging device, and the magnet member 580, of which the position is fixed in the electronic device 400, may be fixed in position relative to each other by the attractive force. Accordingly, the relative positions of the coil of the electronic device 400 and the wireless charging coil 530 of the electronic device 400 may also be fixed. For example, the positions of the coil of the external charging device and the wireless charging coil 530 of the electronic device 400 may be fixed such that the external charging device and the wireless charging coil face each other. As the relative positions of the coil disposed in the external charging device and the wireless charging coil 530 of the electronic device 400 are located within a set range, an electromagnetic induction phenomenon can be smoothly generated between the coil of the external charging device and the wireless charging coil 530. Accordingly, the electronic device 400 can be wirelessly charged by the external charging device.

According to various embodiments, the electronic device 400 may measure a heart rate. For example, the electronic device 400 may measure information related to a user's heart rate through a photoplethysmogrphy method. The photoplethysmogrphy method is a method of measuring a heart rate by emitting light to a part of a user's body 600 (e.g., a wrist). The principle of the photoplethysmogrphy method is as follows. When the heart rate increases due to physical activity, the amount of light reflected by the body and incident on a photoplethysmogrphy device may decrease as the blood flow flowing throughout the body increases. In contrast, when the heart rate decreases, the amount of blood flowing through the whole body may decrease, and accordingly, the amount of light reflected by the body and incident on the photoplethysmogrphy device may increase. As described above, the photoplethysmogrphy device may measure the user's heart rate by comparing the amount of light generated by the photoplethysmogrphy device and the amount of light reflected from the body and incident on the photoplethysmogrphy device.

In an embodiment, the electronic device 400 may include a first biological signal detector 540. The first biological signal detector 540 may include a light emitter 541 and a light receiver 543. Here, the first biological signal detector 540 may be a device for performing the above-described photoplethysmogrphy method, and the first biological signal may be a signal related to the user's heart rate.

In an embodiment, the light emitter 541 of the first biological signal detector 540 may include a light emitting diode (LED). The light generated from the LED of the light emitter 541 may be emitted to the user's body part 600 (e.g., a wrist) that is in contact with the rear cover 510 through the rear cover 510. The light generated from the light emitter 541 may be absorbed in the user's blood, bone, tissue, etc., and some light that is not absorbed may be incident on the light receiver 543 located inside the electronic device 400 again. The degree of absorption of the light generated by the light emitter 541 may be proportional to a change in blood flow due to a heartbeat in a path through which the light passes. For example, a faster heart rate may increase blood flow, which may increase the amount of light absorbed by the blood. In contrast, when the heart rate decreases, blood flow decreases, which may reduce the amount of light absorbed by the blood. The light absorbed again by the light receiver 543 may be received in the state of being subtracted by the amount of light absorbed by blood, skin, tissue, or the like. Accordingly, the user's heart rate may be measured by comparing the amount of light generated from the light emitter 541 and the amount of light incident on the light receiver 543.

In an embodiment, at least a portion of the rear cover 510 may include a light transmissive area. Light generated from the light emitter 541 may pass through the rear cover 510 through the light transmissive area. In addition, the light reflected from the user's body 600 may be transmitted to the light receiver 543 through the light transmissive area.

In an embodiment, referring to FIG. 5B, the light receiver 543 and the light emitter 541 may be disposed adjacent to each other on the second printed circuit board 520. For example, the light receiver 543 may be disposed to surround the light emitter 541. In order to accurately measure the heart rate, of the light generated from the light emitter 541, only the light that is not absorbed by the user's body 600 but reflected should be transmitted to the light receiver 543. When not only the light reflected from the body but also the light generated from the light emitter 541 is directly incident on the light receiver 543 inside the electronic device 400, an inaccurate heart rate may be measured. Accordingly, a light-tight member 590 may be disposed between the light emitter 541 and the light receiver 543 disposed to surround the light emitter 541. Referring to FIG. 6A, the light-tight member 590 may prevent light emitted from the light emitter 541 from leaking between the rear cover 510 and the first magnet 581 and being transmitted to the light receiver 543. Accordingly, the light reflected by the light emitter 541 without being absorbed by the user's body 600 may be transmitted to the light receiver 543.

According to various embodiments, the electronic device 400 may include a second biological signal detector 550. The second biological signal detector 550 may detect a second biological signal. Here, the second biological signal may be a biological signal related to the user's electrocardiogram (ECG). The second biological signal detector 550 may include external electrodes 553 and 554 and internal electrodes 551 and 552.

In an embodiment, the external electrodes 553 and 554 may be formed of a conductive material. The external electrodes 553 and 554 may include a first external electrode 553 and a second external electrode 554. The external electrodes 553 and 554 may be disposed on the first surface (e.g., the surface oriented in the -Y direction with reference to FIG. 6A) of the rear cover 510. Here, the first surface of the rear cover 510 is the outer surface of the rear cover 510, and may be a surface on which the rear cover 510 comes into contact with the user's body 600. The external electrodes 553 and 554 may be disposed on the first surface of the rear cover 510 to come into contact with the user's body 600.

In an embodiment, the internal electrodes 551 and 552 may be formed of a conductive material. The internal electrodes 551 and 552 may include a first internal electrode 551 and a second internal electrode 552. The internal electrodes 551 and 552 may be disposed on the second surface (e.g., the surface oriented in the +Y direction with reference to FIG. 6A) of the rear cover 510. Here, the second surface of the rear cover 510 is an inner surface of the rear cover 510, and may be a surface on which the rear cover 510 faces the second printed circuit board 520. The internal electrodes 551 and 552 may be electrically connected to a contact 560 which will be described later. In an embodiment, referring to FIGS. 6A and 6B, the internal electrodes 551 and 552 may extend from the outer periphery of the second surface of the rear cover 510 to the center to be electrically connected to the contact 560.

According to various embodiments, the external electrodes 553 and 554 and the internal electrodes 551 and 552 may be connected in various ways. In an embodiment, referring to FIGS. 6A and 6B, the rear cover 510 may be provided with a passage 555 penetrating the first surface (e.g., the surface oriented in the -Y direction with reference to FIG. 6A) and the second surface (e.g., the surface oriented in the +Y direction with reference to FIG. 6A) of the rear cover 510. A conductive material for electrically connecting the first external electrode 553 and the first internal electrode 551 to each other and electrically connecting the second external electrode 554 and the second internal electrode 552 to each other may be disposed in the passage 555. In another embodiment, although not illustrated in the drawings, the first external electrode 553 and the first internal electrode 551 may be connected to each other at the outer periphery of the rear cover 510. Similarly, the second external electrode 554 and the second internal electrode 552 may be connected to each other at the outer periphery of the rear cover 510. As the external electrodes 553 and 554 and the internal electrodes 551 and 552 are electrically connected to each other, the second biological signal received by the external electrodes 553 and 554 may be transmitted to the internal electrodes 551 and 552.

According to various embodiments, as described above, the second biological signal detected by the second biological signal detector 550 may be a biological signal related to the user's electrocardiogram. Hereinafter, a process in which the second biological signal is detected through the second biological signal detector 550 including the internal electrodes 551 and 552 and the external electrodes 553 and 554 will be briefly described.

The second biological signal detector 550 may detect an electrocardiogram-related biological signal by measuring an electrical signal when the heart muscle contracts. When the heart muscle contracts or relaxes, an action potential propagates from the heart to the whole body. By attaching electrodes to various parts of the body, a potential difference generated by an electric current caused by the contraction or relaxation of the heart muscle may be obtained. For example, the potential difference may be obtained using the first external electrode 553 of the second biological signal detector 550 and an electrocardiogram electrode (not illustrated) disposed in the electronic device 400. In an embodiment, the electrocardiogram electrode may be disposed on at least one of key input devices 203 and 204 disposed on a side member 206 of the electronic device 400.

The electronic device 400 according to various embodiments disclosed herein may be an electronic device 400 to be worn on a wrist. Of the external electrodes 553 and 554, the first external electrode 553 may come into contact with the user's one wrist 600. In such a state, when the electrocardiogram electrode of the electronic device 400 is brought into contact with the user's opposite finger or wrist, a closed circuit is formed and a potential difference between the wrists according to the heartbeat may be detected. Meanwhile, the second external electrode 554 may serve as a ground electrode. In contrast, in some embodiments, a potential difference generated by a current caused by contraction or relaxation of the heart may be obtained by using the second external electrode 554 and the ECG electrode of the electronic device 400, and the first external electrode 553 may serve as a ground electrode.

According to various embodiments, a voltage change according to time may be detected in the form of a waveform by the first external electrode 553 and the ECG electrode of the electronic device 400. By analyzing the shape (e.g., amplitude, period, or kurtosis) of this waveform, a second biological signal that is a biological signal related to an electrocardiogram may be detected. The operation of the second biological signal detector 550, the sensed second biological signal, and the like may be controlled or processed by a signal processor 570, which will be described later. In some cases, the processor of the electronic device 400 (e.g., the processor 120 of FIG. 1) may operate the second biological signal detector 550, and may process the second biological signal detected from the second biological signal detector 550. The signal processor 570 and the processor of the electronic device 400 (e.g., the processor 120 of FIG. 1) may divide and process instructions required for control and signal processing.

The second biological signal detection of the second biological signal detector 550 described above describes a representative principle of detecting electrocardiogram-related information by using a plurality of electrodes. The second biological signal detector 550 according to various embodiments disclosed herein may detect the user's ECG-related information as the second biological signal in various other methods.

According to various embodiments, the contact 560 may be disposed on the second printed circuit board 520. Referring to FIGS. 6A and 6B, one end of the contact 560 may be disposed on the first surface (e.g., the surface oriented in the -Y direction with reference to FIG. 6A) of the second printed circuit board 520. Here, the first surface of the second printed circuit board 520 may be a surface on which the second printed circuit board 520 faces the rear cover 510. The end of the contact 560 opposite to the one end may be electrically connected to the internal electrodes 551 and 552 disposed on the rear cover 510. The contact 560 may be formed of a conductive material. The contact 560 may be electrically connected to each of the internal electrodes 551 and 552 and the second printed circuit board 520 to electrically connect the internal electrodes 551 and 552 and the second printed circuit board 520 to each other. Accordingly, the second biological signal transferred from the external electrodes 553 and 554 to the internal electrodes 551 and 552 may be transferred to the second printed circuit board 520. Referring to FIGS. 6A and 6B, the contact 560 may be provided to protrude in a direction (e.g., the -Y direction with reference to FIG. 6A) perpendicular to the first surface of the second printed circuit board 520 to be easily connected to the first surface of the second printed circuit board 520 and the internal electrodes 551 and 552.

In an embodiment, a buffer material may be filled inside the contact 560. The contact 560 may act as a buffer between the second printed circuit board 520 and the rear cover 510 when an external force is applied to the electronic device 400. Even when the buffer material is not filled in the contact 560, the contact 560 may be formed of an elastic material, and when an external force is applied to the electronic device 400, the contact 560 may act as a buffer between the second printed circuit board 520 and the rear cover 510.

According to various embodiments, the signal processor 570 may be disposed on the second printed circuit board 520. The signal processor 570 may process the first biological signal detected by the first biological signal detector 540 and the second biological signal sensed by the second biological signal detector 550. For example, the signal processor 570 may convert the first biological signal and/or the second biological signal in an analog form into a signal in a digital form or amplify the first biological signal and/or the second biological signal. The signal processor 570 may be disposed on a second surface (e.g., the surface oriented in the +Y direction with reference to FIG. 6A) opposite to the first surface (e.g., the surface oriented in the -Y direction with reference to FIG. 6A) of the second printed circuit board 520. As described above, as electronic components are disposed on the first and second surfaces of the second printed circuit board 520, the electronic components may be efficiently mounted in the internal space of the electronic device 400.

According to various embodiments, the electronic device 400 may measure the user's body temperature by using the external electrodes 553 and 554 and the internal electrodes 551 and 552 included in the second biological signal detector 550 capable of measuring the second biological signal. Hereinafter, components and a stacked structure for measuring a user's body temperature by using the second biological signal detector 550 will be described.

According to various embodiments, the electronic device 400 may measure the user's body temperature by using the external electrodes 553 and 554 and the internal electrodes 551 and 552 included in the second biological signal detector 550. The external electrodes 553 and 554 and the internal electrodes 551 and 552 are made of a conductive metal, and the metal may be thermally conductive. The external electrodes 553 and 554 and the internal electrodes 551 and 552 may be used as a path through which the user's body temperature is transmitted into the electronic device 400. For example, when the electronic device 400 is a wrist watch-type electronic device 400, as described above, the external electrodes 553 and 554 may be disposed on a first surface (e.g., the surface oriented in the -Y direction with reference to FIG. 6A) of the rear cover 510 to come into contact with the user's wrist 600. Accordingly, the user's body temperature may be transferred to the external electrodes 553 and 554 which are thermally conductive. The user's body temperature transferred to the external electrodes 553 and 554 may be transferred to the internal electrodes 551 and 552 connected to the external electrodes 553 and 554. A temperature sensor 610 that detects a user's body temperature may be disposed inside the electronic device 400. Referring to FIG. 6B, the temperature sensor 610 may be disposed to face at least one of the first internal electrode 551 and the second internal electrode 552 disposed on the second surface (e.g., the surface oriented in the +Y direction with reference to FIG. 6B) of the rear cover 510. The temperature sensor 610 may detect the user's body temperature transferred from the external electrodes 553 and 554 to the internal electrodes 551 and 552.

According to various embodiments, the temperature sensor 610 may include a sensor that measures a temperature in various ways. For example, the temperature sensor 610 may be at least one of a contact-type temperature sensor that comes into contact with a measurement object and responds to a temperature change and a non-contact temperature sensor that detects energy emitted by a measurement object.

In an embodiment, the contact-type temperature sensor may include a thermally variable resistor such as a resistance temperature detector (RTD) or a thermistor. When the temperature sensor 610 is a thermally variable resistor, the electronic device 400 may measure the user's body temperature by using a resistance value that changes depending on the temperature detected by the temperature sensor 610. In some embodiments, the contact temperature sensor may be a thermocouple that detects an electromotive force that changes depending on a temperature change.

In an embodiment, the temperature sensor 610 may include a non-contact temperature sensor. For example, the non-contact temperature sensor may be an infrared temperature sensor that detects a temperature with infrared rays. The infrared temperature sensor may detect a temperature by measuring thermal radiation emitted by a material.

According to various embodiments, the temperature sensor 610 may be electrically connected to the second printed circuit board 520 in various ways. In an embodiment, although not illustrated in the drawings, the temperature sensor 610 may be disposed on the first surface (e.g., the surface oriented in the -Y direction with reference to FIG. 6B) of the second printed circuit board 520 to be directly electrically connected to the second printed circuit board 520. In some embodiments, referring to FIG. 6B, the temperature sensor 610 may be electrically connected to the second printed circuit board 520 via a conductive connecting member 620 disposed between the temperature sensor 610 and the second printed circuit board 520.

According to various embodiments, as the temperature sensor 610 is located closer to the internal electrodes 551 and 552 as a heat conduction path, the conduction path of heat transferred from the internal electrodes 551 and 552 to the temperature sensor 610 may be shortened. Accordingly, the temperature sensor 610 may be located adjacent to the internal electrodes 551 and 552 to efficiently detect heat transferred from the external electrodes 553 and 554 to the internal electrodes 551 and 552.

In an embodiment, the temperature sensor 610 may be in contact with at least one of the internal electrodes 551 and 552 to efficiently detect the user's body temperature. For example, the temperature sensor 610 may be directly disposed on the second printed circuit board 520 such that at least a portion of the temperature sensor is in contact with at least one of the first internal electrode 551 and the second internal electrode 552. In some embodiments, the temperature sensor 610 may be disposed directly on the second printed circuit board 520 to be located adjacent to and not in contact with the internal electrodes 551 and 552.

In an embodiment, as illustrated in FIG. 7A, a connecting member 620 made of a conductive material may be disposed inside the electronic device 400. Here, the connecting member 620 may mean an electronic component such as an interposer, a PCB, an FPCB, or an RFPCB. The connecting member 620 may be disposed between the temperature sensor 610 and the second printed circuit board 520 such that the temperature sensor 610 is disposed adjacent to the internal electrodes 551 and 552. Referring to FIG. 7A, one surface of the connecting member 620 may be disposed on the first surface (e.g., the surface oriented in the - Y direction with reference to FIG. 7A) of the second printed circuit board 520 and electrically connected to the second printed circuit board 520. The surface of the connecting member 620 opposite to the one surface may face the internal electrodes 551 and 552. In some embodiments, since the connecting member 620 is disposed between the temperature sensor 610 and the second printed circuit board 520, the temperature sensor 610 may come into contact with at least one of the first internal electrode 551 and the second internal electrode 552.

The user's body temperature may be transferred into the electronic device 400 through the external electrodes 553 and 554 and the internal electrodes 551 and 552. The external electrodes 553 and 554 and the internal electrodes 551 and 552 may be formed of a conductive metal material. Referring to FIG. 7A, the user's body temperature transferred to the external electrodes 553 and 554 may be transferred to the internal electrodes 551 and 552 connected to the external electrodes 553 and 554. Accordingly, the temperature sensor 610 disposed in contact with the internal electrodes 551 and 552 or disposed at a position adjacent to the internal electrodes 551 and 552 may detect the user's body temperature. For example, the temperature sensor 610 may detect a change in temperature due to the user's body temperature. The operation of the temperature sensor 610 and the change in temperature detected by the temperature sensor 610 may be controlled or processed by the signal processor 570. In some cases, the temperature sensor 610 may be operatively connected with a processor (e.g., the processor 120 of FIG. 1) to operate the temperature sensor 610 or process the user's body temperature detected by the temperature sensor 610. The signal processor 570 and the processor of the electronic device 400 (e.g., the processor 120 of FIG. 1) may divide and process instructions required for control and signal processing.

According to various embodiments, the processor (e.g., the processor 120 of FIG. 1) may display an interface including visual information on the display 220 (e.g., the display module 160 of FIG. 1). In an embodiment, the processor may display an interface for a user's body temperature detected through the temperature sensor 610 on the display 220. In another embodiment, the processor may display an interface related to a user's heart rate measured through the first biological signal detector 540 and/or an interface related to a user's ECG measured through the second biological signal detector 550 on the display 220. In some embodiments, the processor may display at least one of an interface for a user's body temperature, an interface for a user's heart rate, and an interface for a user's electrocardiogram on the display 220. Here, the interfaces are media for communication between the electronic device 400 and the user, and may provide the user with information such as the user's body temperature, heart rate, and electrocardiogram so that the user can check his or her physical condition.

In an embodiment, the processor (e.g., the processor 120 of FIG. 1) may compare a preset control-required value with a value measured through the temperature sensor 610. Here, the control-required numerical value may mean a numerical value (range) obtained by applying a preset ratio to a reference numerical value (range) preset in the processor. When a value measured through the temperature sensor 610 reaches the control-required value, the processor may display the user's status on the display 220. For example, when the value measured through the temperature sensor 610 reaches a predetermined temperature corresponding to the control-required value, the processor may display a warning interface on the display 220 so that the user can take an appropriate action.

In an embodiment, the processor may compare a preset control-required value with the heart rate measured by the first biological signal detector 540. For example, the processor (e.g., the processor 120 of FIG. 1) may display a warning interface on the display 220 when the value measured by the first biological signal detector 540 reaches a heart rate corresponding to the control-required value so that the user can take an appropriate action. In some embodiments, the processor may compare the preset control required value with the electrocardiogram measured by the second biological signal detector 550. For example, the processor may display a warning interface on the display 220 when the ECG measured through the second biological signal detector 550 corresponds to a control-required value (e.g., an irregular waveform) so that the user can take an appropriate action.

According to various embodiments, as illustrated in FIGS. 7A and 7B, a heat insulating member 630 may be disposed between the rear cover 510 and the connecting member 620. The heat insulating member 630 may be disposed to surround at least a portion of the temperature sensor 610. The heat insulating member 630 shields the periphery of the temperature sensor 610 so that the user's body temperature transferred from the external electrodes 553 and 554 to the internal electrodes 551 and 552 can be prevented from being transferred to electronic components other than the temperature sensor 610.

In an embodiment, the heat insulating member 630 may be fixed to be in close contact with the rear cover 510 and the connecting member 620 via an adhesive member such as a bond or adhesive tape. Accordingly, the user's body temperature transferred from the external electrodes 553 and 554 to the internal electrodes 551 and 552 may not be transferred to the electronic components other than the temperature sensor 610.

In an embodiment, the heat insulating member 630 may be disposed between the rear cover 510 and the connecting member 620 to partition the area in which the temperature sensor 610 is disposed. For example, as illustrated in FIG. 7A, the heat insulating member 630 may be disposed between the rear cover 510 and the connecting member 620 to partition' an area in which the temperature sensor 610 is disposed and an area in which the temperature is not disposed with reference to the heat insulating member 630. Hereinafter, the area in which the temperature sensor 610 is disposed will be defined as the inside of the heat insulating member 630. The internal electrodes 551 and 552 disposed on the rear cover 510 may be included inside the heat insulating member 630.

According to various embodiments, as illustrated in FIGS. 7A and 7B, a heat conduction member 640 formed of a heat conductive material may be filled in the inside of the heat insulating member 630 to fill the space between the rear cover 510 and the connecting member 620. The heat conduction member 640 may be a thermal interface material (TIM) excellent in heat conduction efficiency, and may refer to various thermal conductive materials excellent in thermal conductivity. As the heat conduction member 640 comes into contact with the internal electrodes 551 and 552, the user's body temperature transferred from the external electrodes 553 and 554 to the internal electrodes 551 and 552 may be efficiently transferred to the temperature sensor 610. For example, as illustrated in FIGS. 7A and 7B, compared to the case in which the inside of the heat insulating member 630 is filled only with air, the user's body temperature transferred from the external electrodes 553 and 554 to the internal electrodes 551 and 552 may be more efficiently transferred to the temperature sensor 610 when the inside of the heat insulating member is filled with the heat conduction member 640. In various embodiments disclosed herein, the user's body temperature may be transferred from the external electrodes 553 and 554 to the internal electrodes 551 and 552, and may be transferred to the temperature sensor 610 via the heat conduction member 640. As described above, since the periphery of the temperature sensor 610 is shielded by the heat insulating member 630, the user's body temperature may be efficiently transferred to the temperature sensor 610 without being transferred to other electronic components.

According to various embodiments, although not illustrated in the drawings, when the temperature sensor 610 is directly disposed on and connected to the second printed circuit board 520, the heat insulating member 630 may be disposed between the rear cover 510 and the second printed circuit board 520. The heat insulating member 630 may be fixed to the rear cover 510 and the second printed circuit board 520 through an adhesive member such as a bond or adhesive tape to be in close contact with the rear cover 510 and the second printed circuit board 520. The heat conduction member 640 may be filled in the heat insulating member 630 on which the temperature sensor 610 is disposed to fill the space between the rear cover 510 and the second printed circuit board 520.

The shape of the heat insulating member 630 described above is not limited to the shape illustrated in FIG. 7B. Although the heat insulating member 630 illustrated in FIG. 7B has a trapezoidal shape, in another embodiment, the heat insulating member 630 may be formed in various shapes such as a square, a hemispherical shape, a circular shape, and an oval shape.

According to various embodiments, as illustrated in FIGS. 7A and 7B, a metal plate 650 formed of a conductive material may be disposed between the rear cover 510 and the connecting member 620. For example, one surface of the metal plate 650 may be disposed on the first surface (e.g., the surface oriented in the -Y direction with reference to FIG. 7A) of the connecting member 620. The surface of the metal plate 650 opposite to the one surface may be disposed to face the internal electrodes 551 and 552 disposed on the rear cover 510. In an embodiment, the metal plate 650 may be in contact with the internal electrodes 551 and 552.

In an embodiment, the metal plate 650 may be disposed inside the heat insulating member 630 disposed on the first surface (e.g., the surface oriented in the -Y direction with reference to FIG. 7A) of the connecting member 620. For example, referring to FIG. 7B, the metal plate 650 may be surrounded by the heat insulating member 630.

In an embodiment, as illustrated in FIG. 7A, a temperature sensor 610 may be disposed between the outer periphery of the metal plate 650 and the heat insulating member 630. In addition, the above-described heat conduction member 640 may be filled in the space between the outer periphery of the metal plate 650 and the heat insulating member 630. As described above, the user's body temperature may be transferred from the external electrodes 553 and 554 to the internal electrodes 551 and 552. The body temperature transferred to the internal electrodes 551 and 552 may be directly transferred to the temperature sensor 610, and may also be transferred to the metal plate 650 which has a relatively low temperature and is excellent in thermal conductivity. The body temperature transferred to the metal plate 650 may be transferred to the temperature sensor 610 located adjacent to the metal plate 650 via the heat conduction member 640. According to the embodiment disclosed herein, the user's body temperature transferred from the external electrodes 553 and 554 to the internal electrodes 551 and 552 may be transferred to the temperature sensor 610 via the heat conduction member 640 in the internal electrodes 551 and 552, and the body temperature transferred from the internal electrodes 551 and 552 to the metal plate 650 may also be transferred to the temperature sensor 610 via the heat conduction member 640.

In an embodiment, the metal plate 650 may be formed of various materials. The metal plate 650 may be formed of a material that is excellent in electrical conductivity and thermal conductivity. For example, the metal plate 650 may be formed of a material such as stainless use steel (SUS), copper, or aluminum.

According to various embodiments, in order for the second biological signal (e.g., an electrocardiogram) transferred from the external electrodes 553 and 554 to the internal electrodes 551 and 552 to be processed by the signal processor 570, it may be necessary for the internal electrodes 551 and 552 and the second printed circuit board 520 to be electrically connected to each other. As described above, the contact 560 may be disposed between the internal electrodes 551 and 552 and the second printed circuit board 520 such that the internal electrodes 551 and 552 and the second printed circuit board 520 can be electrically connected to each other. The internal electrodes 551 and 552 may be electrically connected to the second printed circuit board 520 via a member other than the contact 560.

In an embodiment, referring to FIGS. 7A and 7B, an adhesive member 660 formed of a conductive material may be applied between the internal electrodes 551 and 552 and the second printed circuit board 520. The adhesive member 660 may electrically connect the internal electrodes 551 and 552 and the second printed circuit board 520 to each other. The adhesive member 660 formed of a conductive material may be formed of various materials. For example, the adhesive member 660 may be formed of an adhesive material that is excellent in electrical conductivity, such as metal epoxy (e.g., Ag epoxy).

According to various embodiments, the adhesive member 660 formed of a conductive material may be applied to the inside of the electronic device 400 in various ways to electrically connect the internal electrodes 551 and 552 and the second printed circuit board 520 to each other.

In an embodiment, referring to FIG. 7B, the connecting member 620 may be provided with a first via hole 670-1 to allow the internal electrodes 551 and 552 and the second printed circuit board 520 to be conductive with each other. An adhesive member 660 formed of a conductive material may be filled in the first via hole 670-1. The internal electrodes 551 and 552 and the second printed circuit board 520 may be electrically connected to each other via the adhesive member 660 applied to the first via hole 670-1.

In an embodiment, referring to FIG. 7B, the metal plate 650 may be provided with a second via hole 670-2. The metal plate 650 may be disposed between the internal electrodes 551 and 552 and the connecting member 620 so that the second via hole 670-2 provided in the metal plate 650 and the first via hole 670-1 provided in the connecting member 620 can be connected to each other. The adhesive member 660 formed of a conductive material may be filled in the second via hole 670-2 provided in the metal plate 650 and the first via hole 670-1 provided in the connecting member 620. The internal electrodes 551 and 552 and the second printed circuit board 520 may be electrically connected to each other via the adhesive member 660. Accordingly, the second biological signal transferred from the external electrodes 553 and 554 to the internal electrodes 551 and 552 may be processed by the signal processor 570.

In another embodiment, although not illustrated in the drawings, the adhesive member 660 formed of a conductive material may be applied elsewhere. For example, the adhesive member 660 formed of a conductive material may be applied between the internal electrodes 551 and 552 and the second printed circuit board 520 to electrically connect the internal electrodes 551 and 552 and the second printed circuit board 520 to each other.

In the foregoing, the components and the stacked structures of the electronic device 400 for measuring a user's body temperature have been described. However, it is not meant to be limited to the components and stacked structures shown in the drawings. At least one of the above-described connecting member 620, the temperature sensor 610, the heat insulating member 630, the heat conduction member 640, the metal plate 650, and the adhesive member 660 formed of a conductive material may be omitted or other components may be added thereto. For example, at least one of the metal plate 650 and the connecting member 620 in the above-described configuration for measuring a body temperature may be omitted.

In some embodiments, the temperature sensor 610 may be disposed directly on the first surface (e.g., the surface oriented in the -Y direction with reference to FIG. 7A) of the second printed circuit board 520, as described above. As the temperature sensor 610 is disposed adjacent to the internal electrodes 551 and 552 or disposed to be in contact with the internal electrodes 551 and 552, the user's body temperature transferred from the external electrodes 553 and 554 to the internal electrodes 551 and 552 can be detected by the temperature sensor 610. In this case, the above-described heat insulating member 630 may be fixed to the rear cover 510 and the second printed circuit board 520 to be in close contact with the rear cover 510 and the second printed circuit board 520. The heat conduction member 640 may be filled in the heat insulating member 630 on which the temperature sensor 610 is disposed to fill the space between the rear cover 510 and the second printed circuit board 520. In addition, the metal plate 650 may be disposed between the second printed circuit board 520 and the rear cover 510. For example, one surface of the metal plate 650 may be disposed on the first surface (e.g., the surface oriented in the -Y direction with reference to FIG. 7A) of the second printed circuit board 520. The surface of the metal plate 650 opposite to the one surface may be disposed to face the internal electrodes 551 and 552. In an embodiment, the metal plate 650 may be electrically connected to the internal electrodes 551 and 552. The metal plate 650 may be surrounded by the heat insulating member 630.

In another embodiment, the rear cover 510, the temperature sensor 610, the second printed circuit board 520 or the rear cover 510, the connecting member 620 on which the temperature sensor 610 is disposed, and the second printed circuit board 520 may be stacked in this order without the metal plate 650.

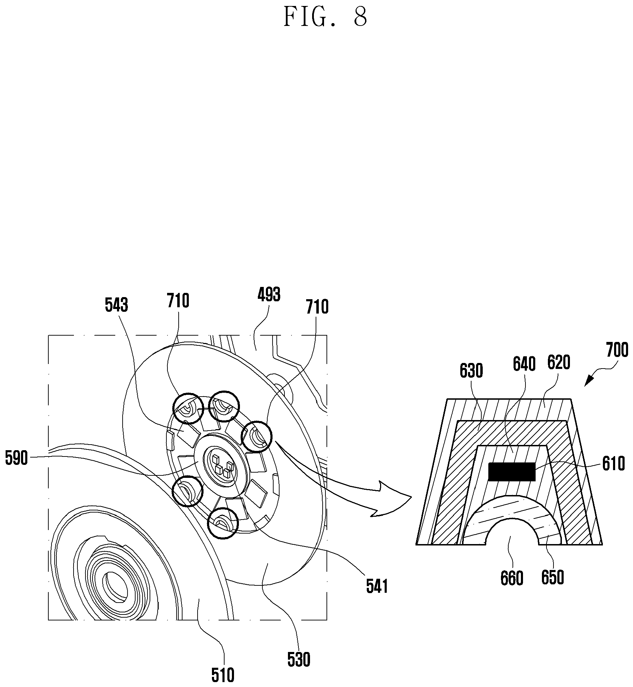

FIG. 8 is an exploded perspective view of the electronic device 400 illustrating the position at which the temperature sensor module 700 according to an embodiment is stacked on the second printed circuit board 520.

Hereinafter, for convenience of description, the above-described connecting member 620, the temperature sensor 610 (e.g., the sensor module 176 of FIG. 1 or the sensor module 211 of FIG. 2), the heat insulating member 630, the heat conduction member 640, the adhesive member 660 formed of a conductive material, and the metal plate 650 will be referred to as a temperature sensor module 700 for measuring a user's body temperature. At least one of the components constituting the temperature sensor module 700 may be omitted or other components may be added thereto.

According to various embodiments, various numbers of temperature sensor modules 700 may be disposed at various positions on the second printed circuit board 520. Referring to FIG. 8, the temperature sensor module 700 may be disposed on at least one of a plurality of seating portions 710 provided on the outer periphery of the second printed circuit board 520. In an embodiment, one or more temperature sensor modules 700 may be disposed on the plurality of seating portions 710 provided on the outer periphery of the second printed circuit board 520. In another embodiment, the temperature sensor module 700 may be disposed in the center of the second printed circuit board 520. In addition, various numbers of temperature sensor modules 700 may be disposed at various positions on the second printed circuit board 520.

An electronic device 400 (e.g., the electronic device 101 of FIG. 1 or the electronic device 200 of FIG. 2) according to various embodiments disclosed herein may include a cover (e.g., the rear cover 510 of FIG. 5A) including a first surface (e.g., the surface oriented in the - Y direction with reference to FIG. 6A) and a second surface (e.g., the surface oriented in the +Y direction with reference to FIG. 6A) opposite to the first surface, a first electrode (e.g., the external electrodes 553 and 554 of FIG. 6A) disposed on the first surface of the cover to be in contact with a user's body, a second electrode (e.g., the internal electrodes 551 and 552 of FIG. 6A) disposed on the second surface of the cover and electrically connected to the first electrode, a printed circuit board (e.g., the second printed circuit board 520 of FIG. 4) disposed to face the second surface of the cover, a temperature sensor (e.g., the sensor module 176 of FIG. 1 or the sensor module 211 of FIG. 2) electrically connected to the printed circuit board and disposed adjacent to the second electrode, and at least one processor (e.g., the processor 120 of FIG. 1) connected to the temperature sensor, wherein the processor may be configured to generate bio-related information based on an electrical signal received through the first electrode and the second electrode, and measure the user's body temperature, which is conducted from the first electrode to the second electrode, via the temperature sensor.

The biometric information may be information related to an electrocardiogram or a heartbeat.

The temperature sensor may be disposed on the printed circuit board such that at least a portion thereof is in contact with the second electrode.

The electronic device may further include a heat insulating member 630 disposed between the cover and the printed circuit board to surround at least a portion of the temperature sensor and partitioning an area in which the temperature sensor is disposed, wherein the second electrode may be included in the area.

The heat insulating member may be disposed between the cover and the printed circuit board to be in close contact with the cover and the printed circuit board.

The electronic device may further include a connecting member 620 disposed between the temperature sensor and the printed circuit board so that the temperature sensor is electrically connected to the printed circuit board, the connecting member being formed of a conductive material, wherein the heat insulating member may be disposed between the cover and the connecting member to be in close contact with the cover and the connecting member.

The electronic device may further include a heat conduction member 640 formed of a heat conductive material and filled in the area in which the temperature sensor is disposed.

The electronic device may further include an adhesive member 660 formed of a conductive material and disposed between the second electrode and the printed circuit board to electrically connect the second electrode and the printed circuit board to each other.

The connecting member may include a first via hole 670-1 formed to allow the second electrode and the printed circuit board to be conductive with each other, and the electronic device may further include an adhesive member 660 formed of a conductive material and filled in the first via hole to electrically connect the second electrode and the printed circuit board to each other.

The electronic device may further include a metal plate 650 disposed between the cover and the connecting member and including a second via hole 670-2 connected to the first via hole, wherein the adhesive member may be filled in the first via hole and the second via hole.

The metal plate may be disposed between the cover and the connecting member to be included in the area in which the temperature sensor is disposed, and the electronic device may further include a heat conduction member 640 formed of a heat conductive material filled in a space between an outer periphery of the metal plate and the heat insulating member.

The electronic device may further include a display module (e.g., the display module 160 of FIG. 1 or the display 220 of FIG. 4) connected to the processor, wherein the processor may be configured to display an interface for the user's body temperature on the display module.

In addition, the processor may be configured to display at least one of the interface for the user's body temperature and an interface for the bio-related information on the display module.

A sensor structure disposed on an electronic device 400 (e.g., the electronic device 101 of FIG. 1 or the electronic device 200 of FIG. 2) according to various embodiments disclosed herein may include a first electrode (e.g., the external electrodes 553 and 554 of FIG. 6A) disposed on a first surface (e.g., the surface oriented in the -Y direction with reference to FIG. 6A) of a cover (e.g., the rear cover 510 of FIG. 5A) of the electronic device to be in contact with a user's body, a second electrode (e.g., the internal electrodes 551 and 552) disposed on a second surface (e.g., the surface oriented in the +Y direction with reference to FIG. 6A) opposite to the first surface of the cover and electrically connected to the first electrode, a printed circuit board (e.g., the second printed circuit board 520 of FIG. 4) disposed to face the second surface of the cover, a temperature sensor 610 (e.g., the sensor module 176 of FIG. 1 or the sensor module 211 of FIG. 2) electrically connected to the printed circuit board and disposed adjacent to the second electrode, and at least one processor (e.g., the processor 120 of FIG. 1) connected to the temperature sensor, wherein the processor may be configured to generate bio-related information based on an electrical signal received through the first electrode and the second electrode, and measure the user's body temperature, which is conducted from the first electrode to the second electrode, by using the temperature sensor.

In addition, the bio-related information may be information related to an electrocardiogram or a heartbeat.

The temperature sensor may be disposed on the printed circuit board such that at least a portion thereof is in contact with the second electrode.

The sensor structure may further include a heat insulating member 630 disposed between the cover and the printed circuit board to surround at least a portion of the temperature sensor and partitioning an area in which the temperature sensor is disposed, wherein the second electrode may be included in the area.

The heat insulating member may be disposed between the cover and the printed circuit board to be in close contact with the cover and the printed circuit board.

The sensor structure may further include a connecting member 620 disposed between the temperature sensor and the printed circuit board so that the temperature sensor is electrically connected to the printed circuit board, the connecting member being formed of a conductive material, wherein the heat insulating member may be disposed between the cover and the connecting member to be in close contact with the cover and the connecting member.

The sensor structure may further include a heat conduction member 640 formed of a heat conductive material and filled in the area in which the temperature sensor is disposed.

The embodiments disclosed in the specification and drawings are provided merely to easily describe the technical features of the disclosure according to the embodiments disclosed herein and to help understanding of the embodiments disclosed herein, and are not intended to limit the scope of the embodiments disclosed herein. Therefore, the scope of the various embodiments disclosed herein should be construed in such a manner that, in addition to the embodiments disclosed herein, all changes or modifications derived from the technical idea of the various embodiments are included in the scope of the various embodiments disclosed herein.

## Claims

1. An electronic device comprising:
a cover including a first surface and a second surface opposite to the first surface;
a first electrode disposed on the first surface of the cover to be in contact with a user's body;
a second electrode disposed on the second surface of the cover and electrically connected to the first electrode;
a printed circuit board disposed to face the second surface of the cover;
a temperature sensor electrically connected to the printed circuit board and disposed adjacent to the second electrode; and
at least one processor connected to the temperature sensor,
wherein the processor is configured to:
generate bio-related information based on an electrical signal received through the first electrode and the second electrode; and
measure the user's body temperature, which is thermally conducted from the first electrode to the second electrode, via the temperature sensor.

2. The electronic device of claim 1, wherein the bio-related information is information related to an electrocardiogram or a heartbeat.

3. The electronic device of claim 1, wherein the temperature sensor is disposed on the printed circuit board such that at least a portion thereof is in contact with the second electrode.

4. The electronic device of claim 1, further comprising:
a heat insulating member disposed between the cover and the printed circuit board to surround at least a portion of the temperature sensor and partitioning an area in which the temperature sensor is disposed,
wherein the second electrode is included in the area.

5. The electronic device of claim 4, wherein the heat insulating member is disposed between the cover and the printed circuit board to be in close contact with the cover and the printed circuit board.

6. The electronic device of claim 4, further comprising:
a connecting member disposed between the temperature sensor and the printed circuit board so that the temperature sensor is electrically connected to the printed circuit board, the connecting member being formed of a conductive material,
wherein the heat insulating member is disposed between the cover and the connecting member to be in close contact with the cover and the connecting member.

7. The electronic device of claim 4, further comprising:
a heat conduction member formed of a heat conductive material that is filled in the area in which the temperature sensor is disposed.

8. The electronic device of claim 1, further comprising:
an adhesive member formed of a conductive material and disposed between the second electrode and the printed circuit board to electrically connect the second electrode and the printed circuit board to each other.

9. The electronic device of claim 6, wherein the connecting member includes a first via hole formed to allow the second electrode and the printed circuit board to be conductive with each other, and
the electronic device further comprises:
an adhesive member formed of a conductive material and filled in the first via hole to electrically connect the second electrode and the printed circuit board to each other.

10. The electronic device of claim 9, further comprising:
a metal plate disposed between the cover and the connecting member and including a second via hole connected to the first via hole,
wherein the adhesive member is filled in the first via hole and the second via hole.

11. The electronic device of claim 10, wherein the metal plate is disposed between the cover and the connecting member to be included in the area in which the temperature sensor is disposed,
the electronic device further comprises:
a heat conduction member formed of a heat conductive material filled in a space between an outer periphery of the metal plate and the heat insulating member.

12. The electronic device of claim 1, further comprising:
a display module connected to the processor,
wherein the processor is configured to:
display an interface for the user's body temperature on the display module.

13. The electronic device of claim 12, wherein the processor is configured to:
display at least one of the interface for the user's body temperature and an interface for the bio-related information on the display module.

14. A sensor structure disposed on an electronic device, the sensor structure comprising:
a first electrode disposed on a first surface of a cover of the electronic device to be in contact with a user's body;
a second electrode disposed on a second surface opposite to the first surface of the cover and electrically connected to the first electrode;
a printed circuit board disposed to face the second surface of the cover;
a temperature sensor electrically connected to the printed circuit board and disposed adjacent to the second electrode; and
at least one processor connected to the temperature sensor,
wherein the processor is configured to:
generate bio-related information based on an electrical signal received through the first electrode and the second electrode; and
measure the user's body temperature, which is conducted from the first electrode to the second electrode, by using the temperature sensor.

15. The sensor structure of claim 14, wherein the bio-related information is information related to an electrocardiogram or a heartbeat.
